# EUROPEAN PATENT APPLICATION

(11) **EP 3 181 072 A1**
(43) Date of publication of application: **21.06.2017**
(21) Application number: 15201106.0
(22) Date of filing: 18.12.2015
(51) Int. Cl.: A61B 17/34

(54) **PNEUMOTHORAX MEDICAL TREATMENT DEVICE**

(71) Applicant: Harder, Paul, Williamsburg, VA 23185 (US); Coakley, Timothy, Virginia Beach, VA 23451 (US); Tyler, James, Cincinnati, OH 45208 (US); Sweatman, Mark, Landrum, SC 29356 (US)
(72) Inventor: Harder, Paul, Williamsburg, VA 23185 (US); Coakley, Timothy, Virginia Beach, VA 23451 (US); Tyler, James, Cincinnati, OH 45208 (US); Sweatman, Mark, Landrum, SC 29356 (US)
(74) Representative: dompatent von Kreisler Selting Werner - Partnerschaft von Patent- und Rechtsanwälten mbB

(57) **Abstract**

The invention provides for an improved medical device used to remove gas and liquid from the plural cavity between the lung and the chest wall in both stationary and mobile applications. The present invention generally comprises a hollow needle with a hollow closed end stylet slidably received in the needle. The stylet is spring-biased to extend out of the needle end as a safety tip but when pressured is moved to a retracted position to allow for insertion through the chest wall. The pressure needed to insert the needle/stylet combination through the chest wall is applied by a housing at the end of the needle which is contoured for easy grip. While not being applied to the chest wall, the stylet comprises a safety tip, which makes the device safe to handle and apply in stationary or mobile applications. Further, the housing optionally includes a one-way valve configured to permit only the passage of air and bodily fluid out of the pleural cavity without a secondary structure needed. The present invention is useful in safely and effectively treating a variety of related conditions including pneumothorax (collapsed lung), tension pneumothorax (collapsed lung pressing on the heart), and pneumohemothorax (collapsed lung with air and bodily fluids in the pleural cavity).

## Description

### BACKGROUND OF THE INVENTION

### Field of the Invention

The present invention relates to a medical device used to remove gas and liquid from the plural cavity between the lung and the chest wall. It is useful in treating a variety of related conditions including pneumothorax (collapsed lung), tension pneumothorax (collapsed lung pressing on the heart), and pneumohemothorax (collapsed lung with air and bodily fluids in the pleural cavity).

### Discussion of Current Methods

A pneumothorax, tension pneumothorax, or pneumohemothorax is caused by the progressive build-up of air within the pleural space or pleural cavity between the chest wall and the lung and is often a result of a laceration to the lung, which allows air and/or bodily fluids to escape from the lung into the pleural cavity.

In mammals, inhalation causes the chest walls to move up and out and the diaphragm, at the lower portion of the thorax, to depress. The lungs, pushed by atmospheric pressure, expand with air to match the pressure gradient. On exhalation, the chest walls move down and in and the diaphragm rises, squeezing the lungs, forcing air from them through the nose and mouth except in the case of a laceration of the lung. The lungs remain inflated, under normal conditions, because of the slight negative pressure in the plural cavity. However, chest trauma allowing air and/or bodily fluids into the pleural space can raise the pressure of the environment, and subsequently collapse the lung.

During respiration, a build up of air and pressure can occur in the pleural space allowing the collapsed lung to push against the pleural wall and may obstruct venous return of deoxygenated blood to the heart. The eventual result of this obstruction from the tension pneumothorax, or pneumohemothorax may result in decreased heart function or traumatic arrest.

Problems with the cannula inserted by the needle are the difficulty in applying sufficient pressure to force the needle with the external cannula into and through the chest wall. When pressure is applied, even a skilled operator may inadvertently puncture the lung with the needle upon entry into the pleural cavity. This would further compound the build-up of air or fluid into the pleural cavity, nullifying the benefits of the application. Further problems can be created after removal of the insertion needle if the cannula is kinked or twisted so as to restrict the passage through the cannula. Also, the open cannula can easily convey non-sterile air into the pleural cavity.

It is desirable to provide a medical device which mitigates the problems associated with the current methods including those involving a plastic cannula. These problems include ease of insertion, inadvertent entry or laceration of the lung during insertion, prevention of any obstruction of the airway and prevention of non-sterile air entering the pleural cavity.

### SUMMARY OF THE INVENTION

The present invention generally comprises a hollow needle with a hollow closed end stylet slidably received in the needle. The stylet is spring-biased to extend out of the needle end as a safety tip but configured to be moved to a retracted position during insertion through the chest wall. The pressure needed to insert the needle/stylet combination through the chest wall is applied by a housing at the end of the needle which is contoured to provide a gripping surface. While not being applied to the chest wall, the stylet comprises a safety tip, which makes the device safe to handle and apply in stationary or mobile applications. Further, the housing optionally includes a one-way valve which is configured without a secondary structure to restrict the passage of air and bodily fluids to only one direction, out of the pleural cavity. There is also an indicator in the housing which shows the extended or retracted position of the stylet in the needle.

One-way valves are known and operate to allow fluid and gas flow in only one direction. A poppet or mushroom valve, as shown in one of the preferred embodiments, is one example of a one-way valve. The portion of the outlet plugged by the valve is known as the seat or the valve seat. In present invention, an application of pressure from inside the housing will cause the valve to lift off of the seat and open allowing fluid to pass out of the housing. Application of pressure from the outside atmosphere will apply a force on the top of the valve, causing the valve to seat over the outlet, closing the valve.

The current methods of treating a pneumothorax are not universally applicable to both on-site and hospital treatments. Operator error is common among the devices currently used, as the force required to puncture the chest wall may lead to further damage to the lung once the needle has reached the extent of the pleural cavity. Devices can also be difficult to secure to the patient without bending and/or kinking because the cannula, tubing or sheath, which is intended to remain inside the patent is small and fits snugly over the puncturing needle, which, to the contrary, is intended to be removed from the patient.

The present invention provides an improved treatment for pneumothorax, tension pneumothorax, and pneumohemothorax; provides a safety mechanism for ease of successful application and safeguards the lung once the device has entered the pleural cavity; provides a visual indication to the operator that after insertion, the stylet has been extended and that the flow between the pleural cavity and the atmosphere is not impeded; provides a device optionally comprising a permanent one-way valve that can be connected to a weak vacuum source; and provides a device, which is relatively simple to manufacture and particularly well adapted for its intended usage.

### BRIEF DESCRIPTION OF THE DRAWINGS

Other advantages of this invention are made apparent in the following descriptions taken in conjunction with the provided drawings wherein are set forth, by way of illustration and example, certain exemplary embodiments of the present invention.
FIG. 1a is a perspective view of a first embodiment in the extended position.
FIG. 1b is a perspective view of a first embodiment in the retracted position.
FIG. 2a is a close-up, cross-sectional view of a first embodiment in the extended position.
FIG. 2b is a close-up, cross-sectional view of a first embodiment in the retracted position.
FIG. 3a is a perspective view of a further embodiment in the extended position.
FIG. 3b is a perspective view of a further embodiment in the retracted position.

### DETAILED DESCRIPTION OF THE INVENTION

As required, detailed embodiments of the present invention are disclosed herein; however, it is to be understood that the disclosed embodiments are merely exemplary of the invention, which may be embodied in various forms. Therefore, specific structural and functional details disclosed herein are not to be interpreted as limiting, but merely as a basis for the claims and as a representative basis for teaching one skilled in the art to variously employ the present invention in virtually any appropriately detailed structure.

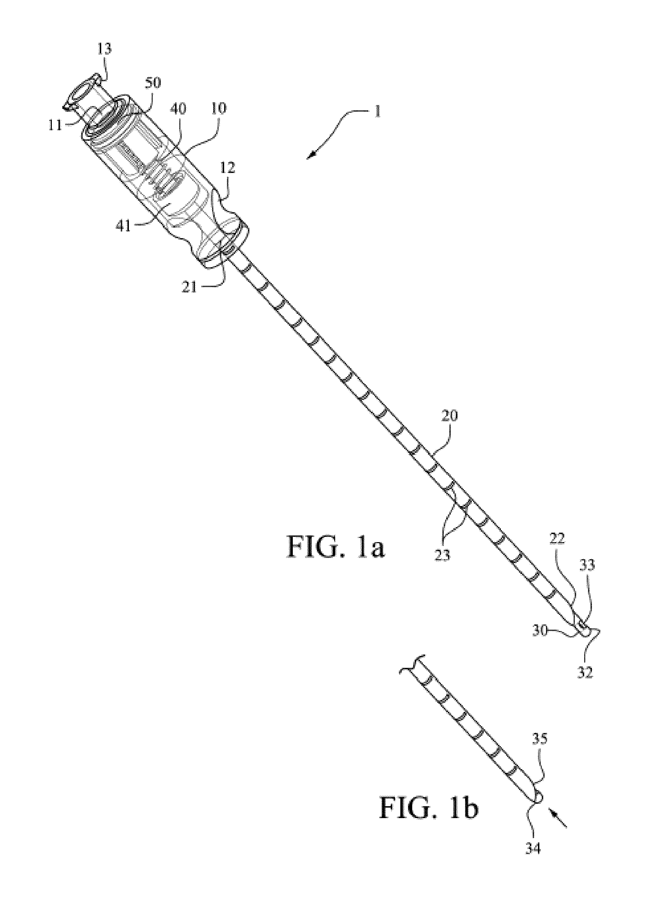

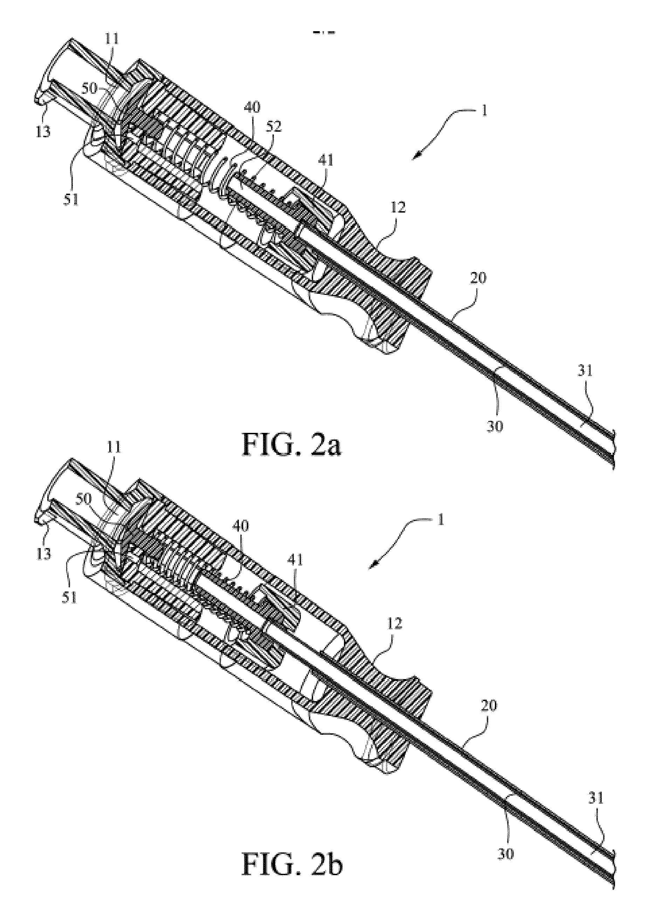

As shown in Figures 1a through 2b, the device 1 generally comprises: A housing 10 with an outlet 11. A hollow needle 20 with one end 21 secured to said housing 10 opposite said housing outlet 11 and another tapered end 22 extending from said housing. A stylet 30 with a hollow inner passage 31, and is slidably received in said needle and moveable between an extended position, in which a closed end 32 of said stylet extends beyond the tapered end of the needle, and a retracted position, in which the closed end of said stylet does not extend beyond said needle. In one embodiment, the stylet includes at least one terminal aperture 33 adjacent the closed end which is fluidly connected to the hollow inner passage.

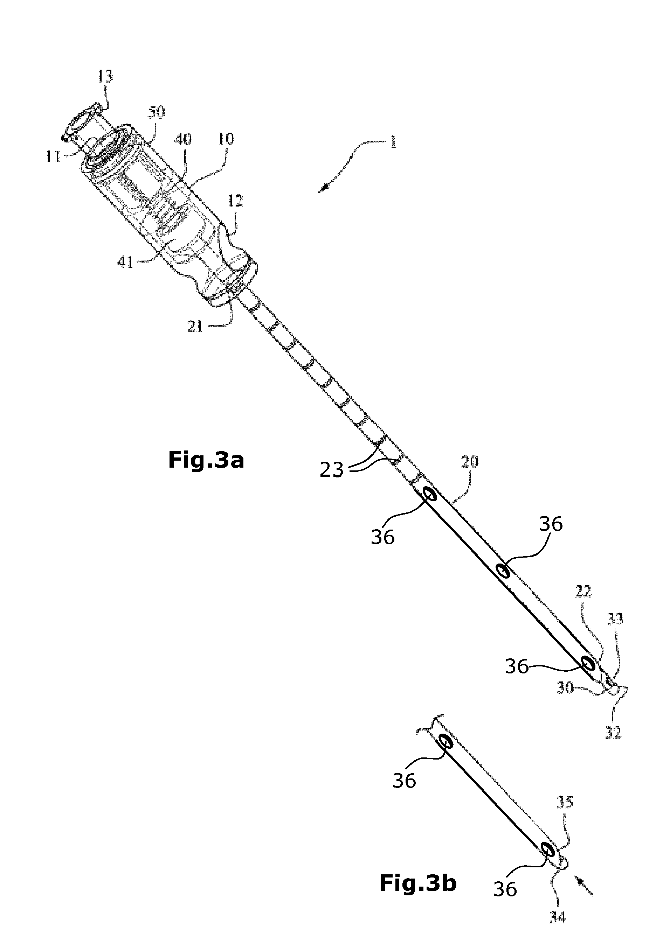

In additional embodiments, as shown in fig. 3a/b, and in addition to the terminal aperture, the stylet includes a plurality of apertures 36 positioned along a length of the stylet 30, where each aperture 36 comprises two substantially aligned openings or holes in the opposing sides of the stylet 30, and where the plurality of apertures 36 are fluidly connected to the hollow inner passage. In further embodiments, the hollow needle also includes a plurality of apertures positioned along a length of the needle, where each aperture comprises two substantially aligned openings or holes in the opposing sides of the needle, and where at least one of the plurality of apertures in the needle is capable of being substantially aligned with at least one of the plurality of apertures 36 in the stylet.

In various embodiments, the apertures of the stylet and/or needle may be any shape, such as circular, oval, square, rectangular, etc. In one embodiment, at least one of the plurality of apertures in the stylet is substantially aligned with at least one of the apertures in the needle when the stylet is in the extended position. In various embodiments, the plurality of apertures of the stylet and the needle are arranged in any desired pattern, as long as at least one of the apertures of the stylet substantially aligns with at least one of the apertures of the needle when the stylet is in the extended position. For instance, the plurality of apertures may be arranged in a straight line along two sides of the stylet and/or needle. Conversely, the plurality of apertures may be arranged such that they are not in a straight line. For example, a first aperture may be located at a position on a side of the needle and/or stylet, and a second or subsequent aperture may be at a position which is rotated by some amount, for example by 90°, from the first position. In addition, in one embodiment, the plurality of apertures of the stylet and/or needle are equidistant from each other, while in another embodiment, the spacing between the plurality of apertures of the stylet and/or needle are not equal. In various embodiments, the maximum size of the aperture is dictated by the width of the needle and/or stylet. In various embodiments, the size of the aperture(s) ranges from about 1.0 mm² to about 5.0 mm².

In one embodiment, the stylet and needle each include three apertures, in addition to the terminal aperture of the stylet, where each aperture comprises two substantially aligned openings or holes in the opposing sides. In one embodiment, the three apertures of the stylet and needle are disposed equidistant from each other, and are positioned in a straight line. In one embodiment, the size of the aperture(s) is about 3.04 mm². In one embodiment, the three apertures of the stylet align with the three apertures of the needle when the stylet is in the extended position.

A spring 40 biases the stylet towards said extended position but can be moved by pressure on the tip to the retracted position when the needle is inserted into the chest wall. Optionally, a one-way valve 50 is located in the housing, and is fluidly connected to said stylet aperture(s) and is configured to only permit flow of gas and/or liquids into the stylet through the aperture(s) and out of the outlet.

The optional one way valve 50, is fluidly connected to the housing by a passage 51 and fluidly connected to the inner passage 31 of the stylet 30 by a connection 52. When the pressure of gas and/or fluid in passage 51 is greater than the pressure on the top of mushroom valve 50, the valve will at least partially open and allow the gas and/or fluid to flow out of the outlet 11. While in this specific embodiment, a mushroom valve is shown, any valve that restricts flow of gas and/or fluid in a single direction, from said stylet aperture to said housing outlet may be used.

In one embodiment, housing 10 with outlet 11 would be shaped to provide a gripping surface 12 configured to permit an operator to exert a force on needle sufficient to penetrate the chest wall of a patient.

In another embodiment, the housing further comprises a visual indication 41, connected to the stylet, for providing a visual indication at the housing for indicating when the stylet is in the retracted state or extended state. Such a visual indication could include, for example, a colored portion of spring, markings to designate extended and retracted position, a lever, a ball and float, etc.

In another embodiment, the hollow needle 20 and the stylet 30 preferably comprise stainless steel. Preferably, the hollow needle 20 and the stylet 30 comprise medical grade material. Preferably, the hollow needle 20 and the stylet 30 and device 1 comprise medical grade material resistant to the temperatures required by sterilization. In one embodiment, the needle length is in the range of about 55 mm to about 90 mm. In one embodiment, the needle is about 63 mm in length. In another embodiment, the needle is about 89 mm in length.

In another embodiment, the device further comprises means for fluidly connecting 13 the housing outlet and the one-way valve to any fluid fitting, where the means for connecting includes a stopcock, a Luer lock, a threaded fitting, slip-tips, fluid couplings, ridged tips, tube fittings, quick-connect fittings, adaptors, nozzles, or combinations thereof.

In another embodiment, wherein the outside surface of said stylet 34 and the inside surface of said hollow needle 35 are configured to slide against one another when moving from the retracted and extended position to effectively scrape and clear said outside surface of said stylet.

In another embodiment, the outer surface of said hollow needle has graduations 23 in intervals to allow an operator to determine the depth of penetration of the device to more accurately and effectively treat all sizes of patients.

Current methods for treating a pneumothorax typically involve removing the entrapped air or fluid from the pleural space. An insertion needle with a surrounding plastic cannula is generally forced into the pleural cavity of the patient through a point between the ribs, determined by feel by an operator. The needle can then be removed leaving only the plastic cannula, which can then be secured to the patient. The exposed end of the tubing may also be connected to a slight vacuum source to pull air and fluids out of the pleural cavity and prevent air from re-entering the pleural space.

### An embodiment of the invention is

a housing with an outlet;
a hollow needle with one end secured to said housing opposite said outlet and the other end extending from said housing, and a plurality of apertures located along a length of the hollow needle,
a stylet having a hollow inner passage, said stylet slidably received in said needle and moveable between an extended position, in which a closed end of said stylet extends beyond the end of said needle, and a retracted position, in which the closed end of said stylet does not extend beyond said needle, said stylet including at least one terminal aperture adjacent said closed end of the stylet and fluidly connected to the hollow inner passage, and a plurality of apertures located along a length of the stylet and fluidly connected to the hollow inner passage;
a spring configured to bias said stylet towards said extended position; and
optionally a one-way valve, located in said housing, and fluidly connected to said stylet aperture, said valve configured to only permit gas flow into the stylet through the aperture and out of said outlet,
where at least one of the plurality of apertures of the needle substantially aligns with at least one of the plurality of apertures of the needle when the stylet is in the extended position
said method comprising:
   applying a downward pressure to the closed end of said stylet to temporarily overcome said spring bias and move said stylet into said retracted position, and continuing said application of pressure as said extended end of said hollow needle passes through the chest wall continuing only until said extended end enters the pleural cavity of said patient,
   wherein gas flow is only permitted into the stylet through the apertures and out of said housing outlet.

In one embodiment, the method further comprises applying an atmospheric vacuum to said housing outlet.

It is to be understood that while certain forms of the present invention have been illustrated and described herein, it is not to be limited to the specific forms or arrangement of parts described and shown.

## Claims

1. A medical device for treating tension pneumothorax in a human patient, said device comprising:
a housing with an outlet;
a hollow needle with one end secured to said housing opposite said outlet and the other end extending from said housing, and a plurality of apertures located along a length of the hollow needle,
a stylet having a hollow inner passage, said stylet slidably received in said needle and moveable between an extended position, in which a closed end of said stylet extends beyond the end of said needle, and a retracted position, in which the closed end of said stylet does not extend beyond said needle, said stylet including at least one terminal aperture adjacent said closed end of the stylet and fluidly connected to the hollow inner passage, and a plurality of apertures located along a length of the stylet and fluidly connected to the hollow inner passage;
a spring configured to bias said stylet towards said extended position; and
optionally a one-way valve, located in said housing, and fluidly connected to said stylet aperture, said valve configured to only permit gas flow into the stylet through the aperture and out of said outlet,
where at least one of the plurality of apertures of the needle substantially aligns with at least one of the plurality of apertures of the needle when the stylet is in the extended position.

2. The medical device of claim 1, wherein said housing is shaped to provide a gripping surface, said gripping surface of said housing configured to permit an operator to exert a force on said needle sufficient to penetrate a chest wall of said human patient.

3. The medical device of claims 1 or 2, further comprising visual indicator means, connected to said stylet, for providing a visual indication at said housing for indicating when the stylet is in the retracted state.

4. The medical device of anyone of claims 1 to 3, further comprising means for fluidly connecting said one-way valve to any fluid fitting or not comprising a one-way valve located in said housing, and fluidly connected to said stylet aperture, said valve configured to only permit gas flow into the stylet through the aperture and out of said outlet.

5. The medical device of anyone of claims 1 to 4, wherein said hollow needle and said stylet are comprised of stainless steel.

6. The medical device of anyone of claims 1 to 5, wherein the outside surface of said stylet and the inside surface of said hollow needle are configured to slide against one another when moving from the retracted and extended position.

7. The medical device of anyone of claims 1 to 6, wherein said stylet comprises two terminal apertures adjacent said closed end of the stylet fluidly connected to the hollow inner passage.

8. The medical device of anyone of claims 1 to 7, wherein the outer surface of said hollow needle is graduated or marked off in intervals.

9. The medical device of anyone of claims 1 to 8, further comprising a container, sterilized and vacuum sealed after said medical device is placed inside.

10. The medical device of anyone of claims 1 to 9, wherein said medical device is comprised of parts capable of resisting temperatures of sterilization.

11. The medical device of anyone of claims 1 to 10, wherein said housing is transparent.

12. The medical device of anyone of claims 1 to 11, wherein the outer diameter of the hollow needle is configured in sizes according to the Birmingham Wire Gauge.

13. The medical device of anyone of claims 1 to 12, wherein said housing is configured to rest upon the chest wall of the patient, to more easily secure said device to said patient.

14. The medical device of anyone of claims 1 to 13, wherein said housing is shaped to provide a gripping surface,
wherein said hollow needle and said stylet are comprised of stainless steel,
wherein the outside surface of said stylet and the inside surface of said hollow needle are configured to slide against one another when moving from the retracted and extended position,
further comprising means for fluidly connecting said one-way valve to any fluid fitting, and
comprising visual indicator means, connected to said stylet, for providing a visual indication at said housing for indicating when the fluid transfer mean is in the retracted state.

15. A medical device for treating tension pneumothorax in a human patient, said device comprising:
a housing with an outlet;
a hollow needle with one end secured to said housing opposite said outlet and the other end extending from said housing, and a plurality of apertures located along a length of the hollow needle;
a fluid transfer means for fluidly connecting the two ends of said hollow needle, said fluid transfer means slidably received in said needle and moveable between an extended position, in which a closed end of said fluid transfer means extends beyond the end of said needle, and a retracted position, in which the closed end of said fluid transfer means does not extend beyond said end of said needle (or needle end), and including at least one terminal aperture adjacent to said closed end, and a plurality of apertures located along a length of the fluid transfer means;
a means for biasing said fluid transfer means towards said extended position; and
optionally a one-way valve means for fluidly connecting said fluid transfer means to said housing outlet for regulating air flow to only permit gas flow into the fluid transfer means through the aperture and out of said housing outlet.
